# EUROPEAN PATENT SPECIFICATION

(11) **EP 2 911 726 B1**
(45) Date of publication and mention of the grant of the patent: **07.06.2017**
(21) Application number: 13818404.9
(22) Date of filing: 21.10.2013
(51) Int. Cl.: A61B 5/15, A61M 5/32

(54) **DISPOSABLE SYRINGE WITH RETRACTABLE NEEDLE**
EINWEGSPRITZE MIT EINZIEHBARER NADEL
SERINGUE JETABLE AVEC AIGUILLE RÉTRACTABLE

(30) Priority: 26.10.2012 IT RM20120514
(43) Date of publication of application: 02.09.2015
(73) Proprietor: Pepe, Mauro, 00167 Roma (IT)
(72) Inventor: Pepe, Mauro, 00167 Roma (IT)
(74) Representative: Fiammenghi, Eva
(86) International application number: PCT/IB2013/059512
(87) International publication number: WO 2014/064600

(56) References cited:
- WO-A1-93/20872
- GB-A- 2 420 713
- US-A- 4 675 005
- US-A- 4 995 874
- US-A- 5 759 177
- US-B1- 6 171 285

## Description

### Field of the art

The present invention refers to a new type of disposable syringe. In particular, the present invention refers to a disposable syringe with retractable needle in which the retraction of the needle occurs by means of particular, characteristic components of the described invention, such to cause the collapse of the needle inside the syringe itself.

### State of the art

Syringes belong to a well-known category of devices that are widely used in the medical field. More in detail, the syringe as it is conceived today is substantially constituted by a hollow cylindrical body inside of which a plunger sealingly slides. A mouth is present at one end of the hollow body; on such mouth, the hollow needle is connected through which the liquid passes that is sucked and/or injected.

With reference to the use intended for the syringes, these can have different capacities. Usually, the capacity varies from 0.5 ml to 100 ml, and for this reason a graduated scale in ml is present on the body of the syringe.

Most of the currently used syringes are made of plastic, have a pre-coupled needle and are generally disposable, unlike the older glass syringes that were used multiple times upon sterilization in an autoclave.

The use of disposable syringes was introduced after numerous accidents had taken place, as a consequence thereof. In particular, the contaminations, accidental puncture and cutting represent the most disturbing main factors of biological risk. Hence, these factors are considered priorities to confront in programs aimed to improve the safety and use conditions of this medical device, both inside hospital structures/clinics and at home.

The use of needles and other sharp devices is indeed of fundamental importance in medical practice. Nevertheless, there is great risk each time a needle or another sharp instrument remains free in the environment and imprudently exposed. This risk involves patients, health workers, family members and guests of the structures. Undoubtedly, the medical devices responsible for the greatest number of cutaneous and percutaneous lesions are those provided with needles. This also occurs due to the wide diffusion of these devices. The accidents usually occur during the use of the device, after use, before disposal, or because the device is left or forgotten in inappropriate places. In addition, most of the people at risk are nurses, doctors, aids, cleaning personnel, training personnel, family members and laboratory technicians.

In particular, the main causes of accidental puncture with needle are represented by the recovering of the needle itself after its use, by the use of improper containers with thin, easily perforable walls and generally by incautious handling and actions during the step of eliminating the needle.

For the purpose of avoiding the frequent repetition of these accidents, over the last few years various technical solutions have been developed and patented that have contributed to reducing the occurrence of accidental punctures with syringe needles. A valid example is represented by the syringes with retractable needle. Nevertheless, these known disposable syringe types of have complex structure, with a high number of components that are costly and hard to use. For such purpose, the present invention provides a new type of disposable syringe with retractable needle that not only prevents the contact of the needle with any one operator but also irreversibly prevents the re-emersion of the needle from the syringe itself.

Indeed the needle, once retracted, could still intentionally or accidentally exit outward from the mouth of the syringe. This occurs by suitably acting on the plunger, for example. The present invention for such purpose provides a mechanism that makes it impossible to reuse the needle after the use of the syringe.

### Description of the invention

Each year, a high number of workers, especially in the medical field, are inadvertently injured or infected with needle punctures. Each infected needle puncture can be potentially lethal. The contaminated needles can transmit many dangerous pathogen agents, transmittable via blood, including hepatitis C and HIV. Most of such lesions are sustained by nurses, doctors and paramedical personnel, but also any other hospital worker is potentially exposed to a considerable risk, such as cleaning personnel, laundry personnel, and other support workers. If one considers the municipal workers who collect solid urban waste or the people and children who come into contact with syringes abandoned on the ground in a wide range of places, such as beaches or parks, the number of potential victims increases exponentially.

The invention described in the present industrial invention application provides a new disposable syringe with retractable needle in which the retraction of the needle, once it has occurred, is irreversible. This important characteristic is made evident due to the presence of an innovative and particular mechanism that is built in the syringe, object of the present invention. This safety mechanism ensures that the retraction of the needle inside the syringe occurs due to the simple collapse of the needle itself, once the blood sample has been drawn or the medicine solution has been injected. The collapse of the needle occurs because the needle itself is removed from the fit support neck, by means of special components of the syringe that is the object of the present invention. In particular, the result that is obtained, following the use of the syringe and upon the retraction of the needle at its interior after use, is a syringe that irreversibly traps the needle at its interior. This arrangement of the needle ensures that even if suitably pressing on the plunger, the outward exit of the needle from the neck is prevented. The present invention also describes a disposable syringe with retractable needle in which the retraction of the needle can possibly be facilitated due to the presence of a spring situated inside the syringe itself.

### BRIEF DESCRIPTION OF THE FIGURES

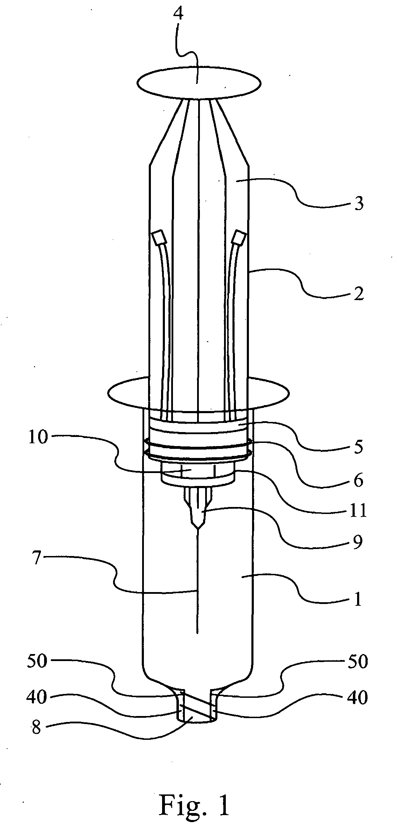

FIGURE 1 shows a side view of the disposable syringe with retractable needle according to the present invention, in which the following are visible: the cylindrical body 1, the plunger 2, the grooves 3 present on the body of the plunger 2, the base 4 of the plunger 2, the head/support 5 of the plunger 2, the seal gasket 6, the needle 7, the neck 8, the support 9 of the needle 7, the housing 10 of the support 9 and the separation element 11.

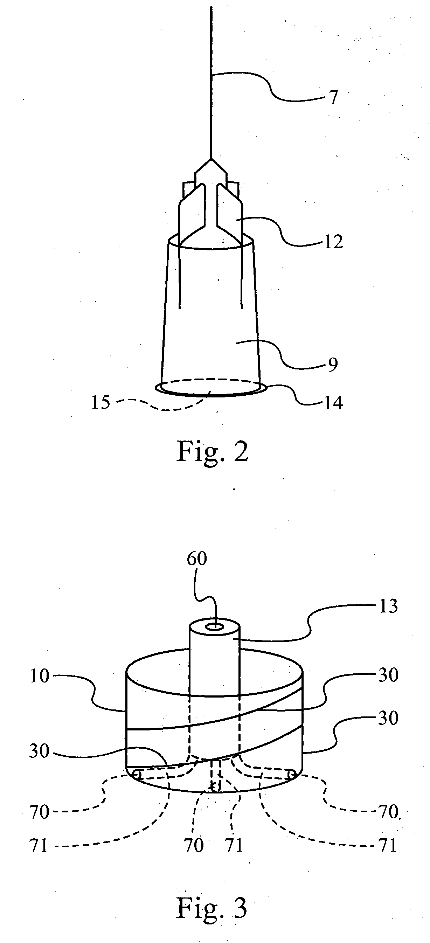

FIGURE 2 shows a view of the support 9 of the needle 7, in which a plurality of fins 12 are visible, along with the circular profile 14 and the open end 15 adapted to be screwed on the housing 10.
FIGURE 3 shows a view of the housing 10 in which the internally hollow cylindrical form of said housing 10 is clearly visible, along with the thread 30 present on the internal surface of the housing 10 and the small cylinder 13, the latter arranged at the center of the housing 10, which has the hole 60 at the center that is connected by means of the channels 71 to the openings 70 arranged at the base of the housing 10. The openings 70 allowing sucking and injecting all the solution present in the cylindrical body 1, by pressing the plunger 2.

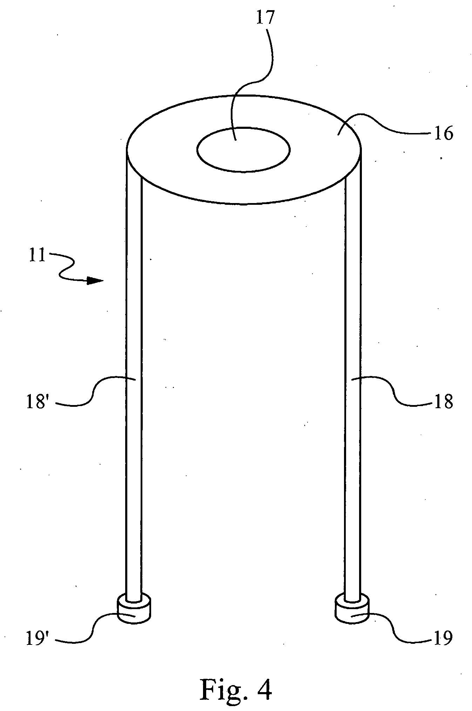

FIGURE 4 shows a detailed perspective view of the separation element 11, in which the following are visible: the discoid base 16 of said separation element 11, the central hole 17 and the small bands 18 and 18' terminating with the respective small cylinders 19 and 19'.

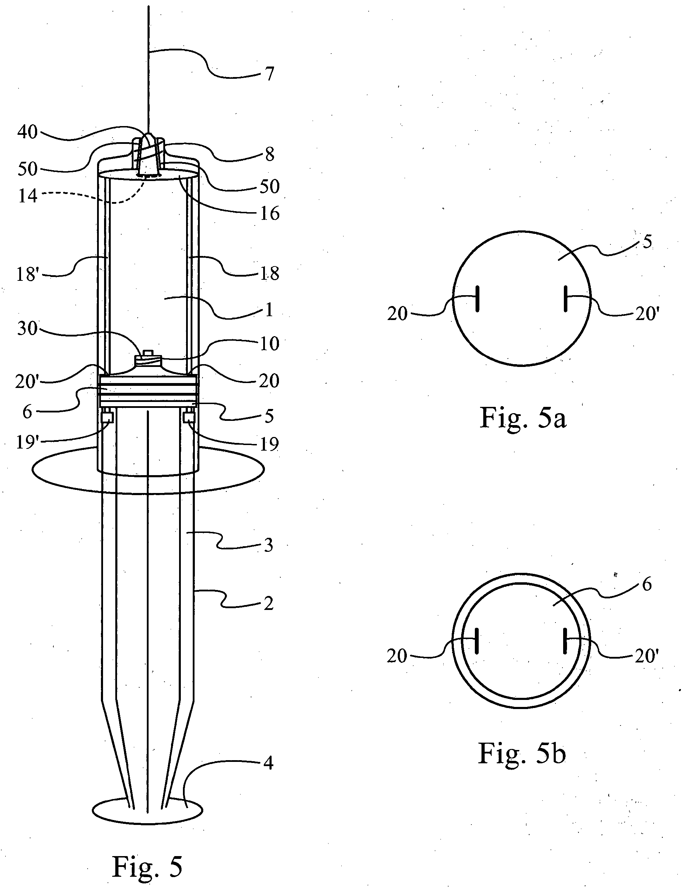

FIGURE 5 shows a view of the disposable syringe with retractable needle according to the present invention, after having carried out the drawing of a liquid. It is observed that the lower part of the head/support 5 of the plunger 2 is in contact with the small cylinders 19 and 19'. It is observed that the thread 30 of the housing 10 is released from the circular profile 14 of the support 9. Said release occurred due to the simple rotation of the plunger 2, integral with the head/support 5 in turn connected with said housing 10, which unscrewed the circular profile 14 from the housing 10. Also observable is a top view of the head/support 5 of the plunger 2 (Fig. 5 (a)) in which the slits 20 and 20' are present that are adapted to respectively allow the traversing of the small bands 18 and 18'. In addition (Fig. 5 (b)), a top view is observed of the seal gasket 6 in which the slits 20 and 20' are visible.

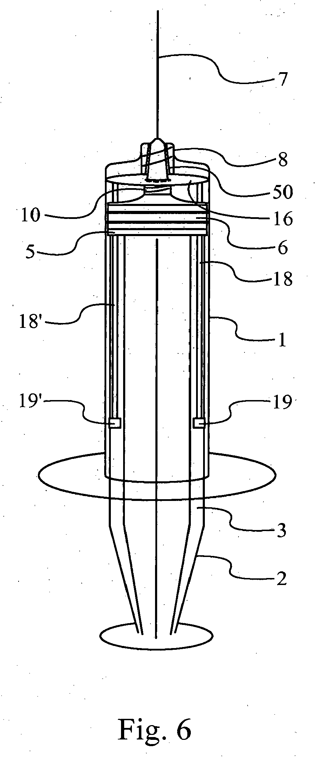

FIGURE 6 shows a view of the disposable syringe with retractable needle according to the present invention, after the injection of a liquid in a patient. The following are observed: the head/support 5 of the plunger 2 and discoid base 16 which is traversed, due to the central hole 17, by the upper part of the support 9 which is fit in the small hollow cylinder 50 arranged at the center of the neck 8.

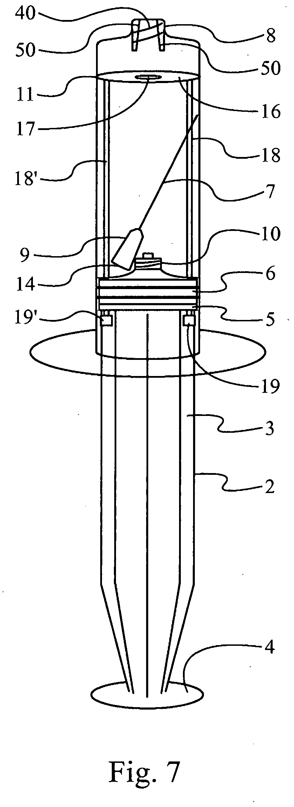

FIGURE 7 shows a side view of the disposable syringe with retractable needle according to the present invention, after its use. The needle 7 and its support 9 are observed after their collapse inside the cylindrical body 1. The collapse occurred due to the traction exerted of the lower part of the head/support 5 on the small cylinders 19 and 19' which in turn, being connected by means of the small bands 18 and 18' to the discoid base 16, have driven the discoid base 16 inside the cylindrical body 1, causing the extraction of the support 9 from the small hollow cylinder 50 of the neck 8.

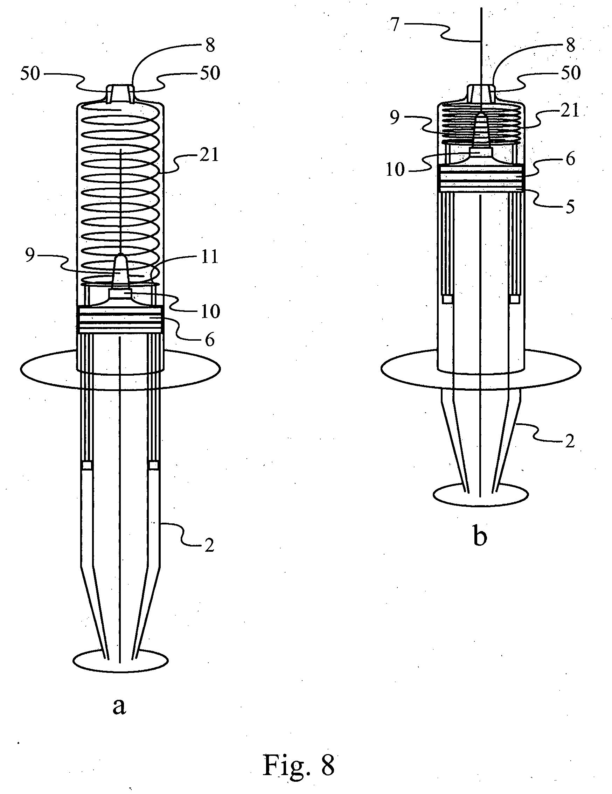

FIGURE 8 shows a perspective view of the disposable syringe with retractable needle according to the present invention, when the spring 21 is present; visible in particular is the case in which said spring 21 is in rest condition (fig. a) and the case in which said spring 21 is compressed (fig. b).

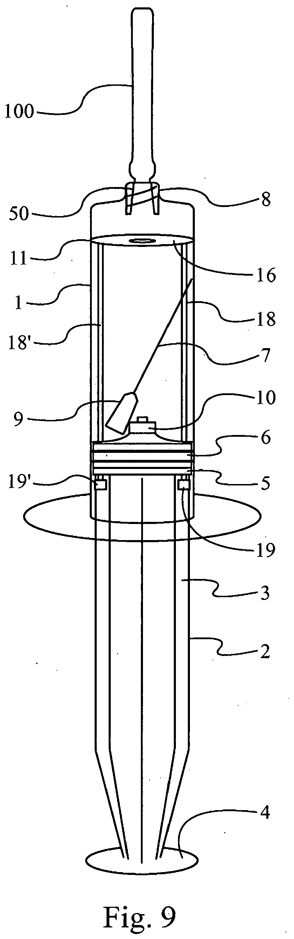

FIGURE 9 shows the syringe, object of the present invention, when the test tube 100 is engaged on the thread 40 inside the neck 8; such test tube 100 also has a mouth equipped with terminal thread. In the drawing, it is observed that the threaded interior of the neck of the test tube 100 is engaged along the thread 40, present on the internal face of the neck 8, in a manner such that the small hollow cylinder 50, arranged at the center of the neck 8 itself, penetrates into the hermetic membrane with which the test tube 100 can be equipped. The test tube 100 can be equipped with a perforable membrane arranged in proximity to the opening in a manner such that by screwing said test tube 100 on the thread 40 one obtains the perforation of said perforable membrane by the small hollow cylinder 50.

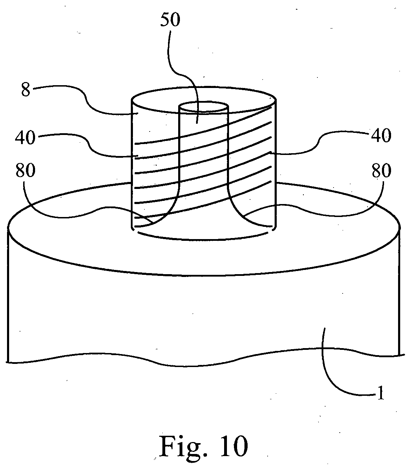

FIGURE 10 is a side view of the neck 8 in which one observes the thread 40 present on its internal surface, the small hollow cylinder 50 arranged centrally with respect to said neck 8 and connected to the cylindrical body 1 by means of its lower end 80.

### Description of the preferred embodiments

The disposable syringe with retractable needle, described in the present invention, is constituted by a cylindrical body 1, by a plunger 2 equipped with a plurality of grooves 3, with a base 4 of the plunger 2 and with a head/support 5 that is integral with a seal gasket 6.

The inventive disposable syringe with retractable needle also comprises a needle 7 and a neck 8 arranged at the upper terminal end of the cylindrical body 1 at the exit of the needle 7.

More in detail, the needle 7 is provided with a support 9, on whose external surface a plurality of fins 12 are present. Before the disposable syringe with retractable needle is used, the needle 7 is situated inside the cylindrical body 1 and is screwed, due to the circular profile 14 of the support 9, inside the housing 10. The support 9 is internally hollow and has the perforated lower end 15 opposite the end from which the needle 7 exits.

Of fundamental importance for the purpose of the present invention is a further element comprised in the disposable syringe with retractable needle, that is the separation element 11 whose function is to make the needle 7 collapse inside the syringe once its use has terminated. The housing 10 carries out the role of support for the support 9 of the needle 7 before using the syringe. Said housing 10 has the shape of a hollow cylinder having a thread 30 obtained on its internal surface and therefore acting as a female thread. Said housing 10 also has, at its interior, a small hollow cylinder 13 which is stably and precisely arranged at the center of the housing 10. The function of the housing 10 is to receive the lower portion of the support 9 of the needle 7. More in detail, the support 9 has, on its lower terminal surface, the circular profile 14 adapted to engage the thread 30, obtained on the internal surface of the housing 10. This screwing allow the progressive insertion of the small cylinder 13 inside the perforated lower end 15 of the support 9. The insertion is complete when the lower part of the support 9, i.e. when the circular profile 14 comes to touch the internal lower portion of the housing 10. The screwing occurs in clockwise sense, the thread being defined right-handed according to current conventions. The aforesaid operation of screwing the support 9 on the housing 10, for the purpose of using the disposable syringe with retractable needle according to the present invention, can be prevented since if the syringe is pre-assembled, the operator will find the needle 7 with its support 9 already screwed in the housing 10.

Alternatively, the assembly operation can be carried out by the user itself. Once assembled as described above, at the time of its use the disposable syringe with retractable needle, according to the present invention, is handled by the operator by initially acting on the plunger 2. In particular, the operator, by exerting a pressure on the base 4 of the plunger 2, will cause the advancement and the progressive exit of the needle 7 from the neck 8. The needle 7 is indeed integral with the plunger 2, given that it is inserted in the support 9 in turn screwed in the housing 10 which in turn is enclosed by the seal gasket 6, it too integral with the plunger 2. More in detail, the seal gasket 6 is integral to the head/support 5 of the plunger 2. Conceptually speaking, given that the base 4 is connected to the plunger 2 and that this is connected by means of the head/support 5 to the seal gasket 6 and to the housing 10 screwed below the support 9 of the needle 7, the advancing of the base 4 will cause the corresponding advancing of the needle 7.

When the needle 7 has completed its travel, i.e. when the upper portion of the support 9 has been inserted inside the small hollow cylinder 50 of the neck 8, the needle 7 will project from said neck 8. The neck 8 at this point will carry out a role of fundamental importance for the functioning of the present invention. In particular, the neck 8 has, analogous to the housing 10, the form of a hollow cylinder having the thread 40 on the internal surface. Said neck 8 also has, at its interior, a small hollow cylinder 50 which is stably and precisely arranged at the center of the neck 8. The neck 8 therefore has a structure entirely analogous to that of the housing 10. Therefore, by pressing on the base 4 and having fit the upper portion of the support 9 inside the small hollow cylinder 50, making the plunger 2 rotate around its axis in counter-clockwise sense, the unscrewing will be caused of the circular profile 14 from the thread 30 of the housing 10.

This unscrewing disengages the housing 10 from the support 9, ensuring that the support 9, to which the needle 7 is joined, and the entire separation element 11 remain fit inside the small hollow cylinder 50. The housing 10, the head/support 5 of the plunger 2, and the seal gasket 6, being released from the support element 9, are free to slide inside the cylinder 1, given that now they are only connected to the base 4 of the plunger 2. By acting on the base 4 of the plunger 2, they can therefore move the head/support 5 and the seal gasket 6 forward or back as desired inside the cylinder 1, without the support 9 and the needle 7 being freed from the small hollow cylinder 50.

The fins 12 stably fit the support 9 inside the small hollow cylinder 50 of the neck 8, therefore, when the operator rotates the plunger 2 in anti-clockwise sense, the circular profile 14 of the support 9 will consequently be unscrewed from the thread 30 of the housing 10. In this configuration, the disposable syringe with retractable needle according to the present invention is ready for its use. The operator can draw and/or inject a specific liquid substance due to the free sliding of the head/support 5 and the seal gasket 6 which are now constrained to the single plunger 2. Therefore, both in the case in which a blood sample is drawn from the patient's body and when an injection is made into the patient's body, the syringe will have the needle 7 in a configuration stably projecting from the neck 8. Once the drawing and/or injection operation has terminated, the retraction and the collapse of the needle inside the cylindrical body 1 will occur due to the innovative presence of the separation element 11.

Said separation element 11 is constituted by a discoid base 16 having one hole 17 and two small bands 18 and 18' each terminating in two respective small cylinders 19 and 19'.

The separation element 11 is positioned inside the disposable syringe with retractable needle, in a manner such to have the discoid base 16 arranged above the surface of the seal gasket 6 and traversed by the upper portion of the support 9. Said separation element 11 has the small bands 18 and 18', with length equal to the graduated scale of the cylindrical body 1, passing through the slits 20 and 20' present on the seal gasket 6 and on the head/support 5 of the plunger 2. In addition said small bands 18 and 18' are extended along the grooves 3 of the plunger 2. The hole 17 has a diameter such to receive and surround the support 9 of the needle 7. The important function of the separation element 11 is completed after drawing a liquid, for example after drawing a blood sample, and after drawing followed by an injection, for example a drawing and an injection of a medicine. In other words, the separation element 11 only becomes operative at the end of the use of the syringe, object of the present invention.

In the particular case of the blood drawing, the syringe that is the object of the present invention will have a configuration in which the needle 7 stably projects outside the neck 8 and the support 9 will have the fins 12 fit against the internal surface of the small cylinder 50, the support 9 inserted inside the hole 17 of the separation element 11. The gasket 6 will be situated inside the cylindrical body 1 at a specific graduated notch present on the surface of the cylindrical body 1, indicating the quantity of blood drawn. The small bands 18 and 18', starting from the discoid base 16, are extended, continuing inside the cylindrical body 1 and through the seal gasket 6 and the head/support 5 by means of the passage through the corresponding slits 20 and 20'. The small cylinders 19 and 19' are also situated inside the cylindrical body 1 but at the opposite side with respect to the discoid base 16, directed towards the base 4 of the plunger 2. The length of the two small bands 18 and 18' is slightly less than the length of the cylindrical body 1. This ensures that when the operator completely retracts the plunger, i.e. beyond the limit set by the graduated scale, the seal gasket 6 and more in detail the head/support 5, moving back encounter as obstacle the two small cylinders 19 and 19'. This occurs because the seal gasket 6 and the head/support 5 of the plunger 2 slide along the small bands 18 and 18' during the retraction of the plunger 2 itself. Said small cylinders 19 and 19' are sized in a manner such to not pass through the slits 20 and 20'. The consequence of this contact ensures that by continuing to further retract the plunger, the head/support 5 drives the two small cylinders 19 and 19' therewith, as well as the discoid base 16 that surrounds the support 9. The result of this moving back ensures that the support 9 of the needle 7 comes to drive the entire element 11 and that it collapses inside the cylindrical body 1, all of this without the seal gasket 6 exiting from the lower part of the cylindrical body 1. The separation element 11 has a hole 17 having diameter such to receive and allow the passage of the front part of the support 9 of the needle 7, whereas the circular profile 14 of the support 9, having greater diameter with respect to said hole 17, will remain positioned below said hole 17. At the end of a blood drawing, the presence of the needle 7 inside the cylindrical body 1 is irrelevant for the purposes of the blood test, given that the needle is soiled only with the blood of the patient. In addition, the transfer of the blood contained in the cylindrical body 1 inside a hermetic test tube 100 will occur by using a test tube 100 whose open end is sealed by a perforable membrane. The blood or the biological liquid drawn with the syringe, object of the present invention, will then be sucked by the vacuum present inside the test tube 100, by simply screwing the threaded end of the test tube 100 on the thread 40 present on the internal face of the neck 8. This screwing causes the advancing of the end of the test tube 100 inside the neck 8 itself, causing the perforation of the membrane by the small hollow cylinder 50 arranged at the center of the neck 8. Given that the small cylinder 50 is in direct connection with the cylindrical body 1, the content of the entire cylindrical body 1 will immediately pass inside the test tube 100.

The collapse mechanism 11 of the needle 7 and of its support 9 inside the cylindrical body 1 is analogous to the aforesaid also in the case in which an injection is executed. The only difference is that after having executed the injection, the cylindrical body 1 will be empty and the needle 7 will be simply made to collapse inside the cylindrical body 1 due to the action of the base 4, which by extracting the head/support 5 beyond the limit of the graduated scale, present on the body itself of the syringe, will cause the coupling of the two small discs 19 and 19', which by driving the discoid base 16 towards cylindrical body 1 will cause the moving back of the support 9 and the needle 7. The housing 10 is equipped with an internally hollow cylindrical form and the thread 30 present on the internal surface of the housing 10 is adapted to house the circular profile 14 of the support 9. The small cylinder 13, arranged at the center of the housing 10, has at the center the hole 60 connected by means of the channels 71 to the openings 70 arranged at the base of the housing 10. The openings 70, by connecting the interior of the cylindrical body 1 with the needle 7, allow injecting all of the solution present in the cylindrical body 1 itself.

In another embodiment, the syringe described in the present invention is provided with a further element and more in detail with the spring 21, present inside the cylindrical body 1.

The presence of the spring 21, both in the case of drawing and injection, serves to facilitate or automate the mechanism of retraction of the needle 7. Indeed, the operator will merely have to suitably push the plunger 2 in the direction of the neck 8, consequently contracting the spring 21. The retraction of the needle will occur after the spontaneous extension of the spring 21.

In all embodiments thereof, the disposable syringe with retractable needle described in the present invention is constituted by elements that can be made of metallic or polymeric material.

## Claims

1. Syringe with retractable needle, **characterized by** a cylindrical body (1) equipped with a neck (8), with a needle (7) installed on the support (9) whose lower margin is equipped with at least one circular profile (14) adapted to be screwed and unscrewed, by respectively rotating the plunger (2) in clockwise and counter-clockwise sense, in the suitable housing (10) positioned at the center of the seal gasket (6) in turn connected to the head/support (5) of the plunger (2), said plunger (2) being equipped with a base (4) and with a plurality of grooves (3) adapted to house the small bands (18, 18') of the separation element (11) in whose hole (17) the fins (12) of the support element (9) are inserted in a manner such that, once said fins (12) are fit inside the small hollow cylinder (50) of the neck (8) and once, due to the counter-clockwise rotation of the plunger (2), the circular profile (14) is unscrewed from the housing (10), by retracting said plunger (2) one obtains only the moving back of the head/support (5) and the seal gasket (6), a retraction which if extended beyond the limit of the graduated scale placed on the cylindrical body (1) will cause the contact of the terminal small cylinders (19, 19') of the small bands (18, 18') with the lower face of the head/support (5), causing the disengagement of the fins (12) of the support (9) from the interior of the small hollow cylinder (50) of the neck (8), making the needle (7) fall inside the cylindrical body (1), both said seal gasket (6) and said head/support (5) having at least two hermetic slits (20, 20') through which the small bands (18, 18') can respectively slide, in a manner so as to prevent liquid leakage when the plunger (2) is brought forward or back inside the cylindrical body (1).

2. Disposable syringe with retractable needle according to the preceding claim, **characterized in that** the housing (10) is integral and inserted at the center of the seal gasket (6) and has the shape of a hollow cylinder on whose internal surface the thread (30) is present, adapted to engage the circular profile (14), and **in that** said housing (10) has a second small cylinder (13) at its center.

3. Disposable syringe with retractable needle according to the preceding claims, **characterized in that** the support (9) of the needle (7) is internally hollow, so as to receive, at its interior, the small cylinder (13) in the open end (15) when the circular profile (14) is screwed on the thread (30), and said support (9) having a plurality of fins (12) adapted to be fit on the internal face of the small hollow cylinder (50) of the neck (8) when the plunger (2) is completely pressed inside the cylindrical body (1).

4. Disposable syringe with retractable needle according to the preceding claims, wherein the separation element (11) is arranged inside the cylindrical body (1) in a manner so as to be traversed, due to the hole (17), by the fins (12) and by the needle (7) of the support (9) and said separation element (11) being constituted by a discoid base (16) having said hole (17) and by at least two small bands (18, 18') terminating with two respective small cylinders (19, 19') in a manner such that said small bands (18, 18') are extended along the grooves (3) of the plunger (2).

5. Disposable syringe with retractable needle according to the preceding claims, **characterized in that** the neck (8) of the cylindrical body (1) has, on its internal surface, a thread (40) adapted to allow the screwing of a sterile test tube (100) equipped with a corresponding thread, in a manner such that the small hollow cylinder (50) connected on the lower part to said neck (8), being placed centrally with respect to said neck (8), is inserted inside the test tube (100) after having perforated the membrane which seals the open end thereof.

6. Disposable syringe with retractable needle according to the preceding claims, **characterized in that** the separation element (11) has a hole (17) having diameter such to receive and allow the passage of the fins (12) of the support (9) of the needle (7) whereas the circular profile (14) of the support (9), having greater diameter with respect to said hole (17), will remain positioned below said hole (17).

7. Disposable syringe with retractable needle according to the preceding claims, **characterized in that** the small cylinders (19, 19') have a size considerably greater than the slits (20, 20'), such that they cannot pass inside said slits (20, 20').

8. Disposable syringe with retractable needle according to the preceding claims, comprising a spring (21) arranged inside the cylindrical body (1) and above the seal gasket (6), adapted to facilitate the retraction of the plunger (2) which causes the consequent moving back of the seal gasket (6), which by driving the small cylinders (19, 19'), prevented by their size from passing into the slits (20, 20'), causes the liberation of the fins (12) of the support (9) from the interior of the small cylinder (50), causing the falling of the needle (7) inside the cylindrical body (1).

9. Disposable syringe with retractable needle according to the preceding claims, **characterized in that** the small bands (18, 18') of the separation element (11) have a lower length than the length of the cylindrical body (1), similar to the width of the graduated scale arranged on said cylindrical body (1) in a manner such that by moving the head/support (5) back beyond the end of said graduated scale, one obtains the separation of the fins (12) of the support (9) from the interior of the small hollow cylinder (50) before said head/support (5) exits outward from the open end of the cylindrical body (1).

10. Disposable syringe with retractable needle according to the preceding claims, **characterized in that** it has the small cylinder (13) centrally equipped with the hole (60) and said hole (60) being in contact, by means of a plurality of holes (70) and channels (71), with the cylindrical body (1).

## Patentansprüche

1. Spritze mit einziehbarer Nadel, **gekennzeichnet durch** einen zylindrischen Körper (1), der mit einem Hals (8) versehen ist, mit einer Nadel (7), die in einen Träger (9) eingesetzt ist, dessen unterer Rand mit mindestens einem kreisförmigen Profil (14) versehen ist, das dafür ausgelegt ist, durch entsprechendes Drehen eines Kolbens (2) im Uhrzeigersinn und entgegen dem Uhrzeigersinn in einem passenden Gehäuse (10), das in der Mitte einer Dichtung (6) angeordnet ist, die ihrerseits mit einem Kopf/Träger (5) des Kolbens (2) verbunden ist, an- und abgeschraubt zu werden, wobei der Kolben (2) mit einer Basis (4) und mit mehreren Rillen (3) versehen ist, die dafür ausgelegt sind, kleine Streben (18, 18') eines Trennelements (11) aufzunehmen, das ein Loch (17) aufweist, in das Rippen (12) des Trägerelements (9) auf solche Weise eingeführt werden, dass, nachdem die Rippen (12) in einen kleinen Hohlzylinder (50) des Halses eingeführt worden sind und nachdem wegen der Drehung des Kolbens (2) entgegen dem Uhrzeigersinn das kreisförmige Profil (14) vom Gehäuse abgeschraubt worden ist, durch Zurückziehen des Kolbens (2) nur eine Zurückbewegung des Kolbens/Trägers (5) und der Dichtung (6) erhalten wird, ein Zurückziehen, wenn es über die Grenze der an dem zylindrischen Körper (1) angebrachten Gradskala hinaus durchgeführt wird, einen Kontakt von endständigen kleinen Zylindern (19, 19') der kleinen Streben (18, 18') mit der unteren Fläche des Kopfes/Trägers (5) bewirkt, wodurch eine Lösung der Rippen (12) des Trägers (9) aus dem Inneren eines kleinen Hohlzylinders (50) des Halses (8) bewirkt wird, wodurch die Nadel (7) in den zylindrischen Körper (1) fällt, wobei sowohl die Dichtung (6) als auch der Kopf/Träger (5) mindestens zwei hermetische Schlitze (20, 20') aufweisen, durch die hindurch die kleinen Streben (18, 18') jeweils auf eine Weise gleiten können, dass ein Austreten von Flüssigkeit verhindert wird, wenn der Kolben (2) innerhalb des zylindrischen Körpers nach vorne oder nach hinten gebracht wird.

2. Einwegspritze mit einziehbarer Nadel nach dem vorangehenden Anspruch, **dadurch gekennzeichnet, dass** das Gehäuse (10) in die Mitte der Dichtung (6) integriert und eingeführt ist und die Form eines Hohlzylinders aufweist, an dessen Innenfläche ein Gewinde (30) vorhanden ist, das dafür ausgelegt ist, mit dem kreisförmigen Profil (14) in Eingriff zu kommen, und dadurch, dass das Gehäuse (10) in seiner Mitte einen zweiten kleinen Zylinder (13) aufweist.

3. Einwegspritze mit einziehbarer Nadel nach den vorangehenden Ansprüchen, **dadurch gekennzeichnet, dass** der Träger (9) der Nadel (7) innen hohl ist, so dass er in seinem Inneren den kleinen Zylinder (13) im offenen Ende (15) aufnehmen kann, wenn das kreisförmige Profil (14) auf das Gewinde (30) geschraubt wird, und wobei der Träger (9) mehrere Rippen (12) aufweist, die dafür ausgelegt sind, an die Innenfläche des kleinen Hohlzylinders (50) des Halses (8) gepasst zu werden, wenn der Kolben (2) vollständig in den zylindrischen Körper (1) gepresst wird.

4. Einwegspritze mit einziehbarer Nadel nach den vorangehenden Ansprüchen, wobei das Trennelement (11) innerhalb des zylindrischen Körpers (1) auf solche Weise angeordnet ist, dass es aufgrund des Loches (17) von den Rippen (12) und von der Nadel (7) des Trägers (9) quer verlagert wird, und dass das Trennelement (11) von einer scheibenförmigen Basis (16), die das Loch (17) aufweist, und von mindestens zwei kleinen Streben (18, 18') gebildet wird, die in zwei entsprechenden kleinen Zylindern (19, 19') enden, auf solche Weise, dass sich die kleinen Streben (18, 18') entlang der Rillen (3) des Kolbens (2) erstrecken.

5. Einwegspritze mit einziehbarer Nadel nach den vorangehenden Ansprüchen, **dadurch gekennzeichnet, dass** der Hals (8) des zylindrischen Körpers (1) an seiner Innenfläche ein Gewinde (40) aufweist, das dafür ausgelegt ist, das Anschrauben eines sterilen Teströhrchens (100), das mit einem entsprechenden Gewinde versehen ist, auf solche Weise zu gestatten, dass der kleine Hohlzylinder (50), der am unteren Teil mit dem Hals (8) verbunden ist und der mittig in Bezug auf den Hals (8) angeordnet ist, in das Teströhrchen (100) eingeführt wird, nachdem er die Membran durchstoßen hat, die dessen offenes Ende abdichtet.

6. Einwegspritze mit einziehbarer Nadel nach den vorangehenden Ansprüchen, **dadurch gekennzeichnet, dass** das Trennelement (11) ein Loch (17) aufweist, das einen solchen Durchmesser aufweist, dass es die Rippen (12) des Trägers (9) der Nadel (7) aufnehmen und durchgleiten lassen kann, wohingegen das kreisförmige Profil (14) des Trägers (9), das einen größeren Durchmesser aufweist als das Loch (17), unter dem Loch (17) angeordnet bleibt.

7. Einwegspritze mit einziehbarer Nadel nach den vorangehenden Ansprüchen, **dadurch gekennzeichnet, dass** die kleinen Zylinder (19, 19') eine Größe aufweisen, die diejenige der Schlitze (20, 20') bei weitem übertrifft, so dass sie nicht in die Schlitze (20, 20') gleiten können.

8. Einwegspritze mit einziehbarer Nadel nach den vorangehenden Ansprüchen, eine Feder (21) umfassend, die innerhalb des zylindrischen Körpers (1) und oberhalb der Dichtung (6) angeordnet ist und die dafür ausgelegt ist, das Zurückziehen des Kolbens (2) zu erleichtern, was die daraus folgende Zurückbewegung der Dichtung (6) bewirkt, die durch Antreiben der kleinen Zylinder (20, 20'), die durch ihre Größe daran gehindert werden, in die Schlitze (20, 20') zu gleiten, die Freigabe der Rippen (12) des Trägers (9) aus dem Inneren des kleinen Zylinders (50) bewirkt, wodurch bewirkt wird, dass die Nadel (7) in das Innere des zylindrischen Körpers (1) fällt.

9. Einwegspritze mit einziehbarer Nadel nach den vorangehenden Ansprüchen, **dadurch gekennzeichnet, dass** die kleinen Streben (18, 18') des Trennelements (11) eine geringere Länge aufweisen als die Länge des zylindrischen Körpers (1), ähnlich der Breite der Gradskala, die am zylindrischen Körper (1) angeordnet ist, auf eine solche Weise, dass durch Zurückbewegen des Kopfes/Trägers (5) über das Ende der Gradskala hinaus die Trennung der Rippen (12) des Trägers (9) vom Inneren des kleinen hohlen Zylinders (50) bewirkt wird, bevor der Kopf/Träger aus dem offenen Ende des zylindrischen Körpers (1) austritt.

10. Einwegspritze mit einziehbarer Nadel nach den vorangehenden Ansprüchen, **dadurch gekennzeichnet, dass** der kleine Zylinder (13) mittig mit dem Loch (60) versehen ist und das Loch (60) mittels mehrerer Löcher (70) und Kanäle (71) mit dem zylindrischen Körper (1) in Kontakt steht.

## Revendications

1. Seringue avec aiguille rétractable, **caractérisée par** un corps cylindrique (1), **caractérisée par** un corps cylindrique (1) équipé d'un col (8), avec une aiguille (7) installée sur le support (9) dont le bord inférieur est équipé d'au moins un profilé circulaire (14) apte à être vissé et dévissé, par une rotation respective du poussoir (2) dans le sens des aiguilles d'une montre et dans le sens inverse des aiguilles d'une montre, dans le boîtier approprié (10) placé au centre du joint d'étanchéité (6), lui-même relié à la tête/support (5) du poussoir (2), ledit poussoir (2) étant équipé d'une base (4) et de plusieurs rainures (3) aptes à loger les petites bandes (18, 18') de l'élément de séparation (11) dans le perçage (17) duquel les ailettes (12) de l'élément de support (9) sont introduites de telle sorte que, une fois que les ailettes (12) sont calées dans le petit cylindre creux (50) du col (8) et une fois qu'en raison de la rotation du poussoir (2) dans le sens inverse des aiguilles d'une montre le profilé circulaire (14) est dévissé du boîtier (10), en tournant le poussoir (2) on obtienne seulement le recul de la tête/support (5) et du joint d'étanchéité (6), une rétraction qui, si elle est étendue au-delà de la limite de l'échelle graduée placée sur le corps cylindrique (1), provoquera le contact des petits cylindres d'extrémité (19, 19') des petites bandes (18, 18') avec la face inférieure de la tête/support (5), provoquant le désaccouplement des ailettes (12) du support (9) par rapport à l'intérieur du petit cylindre creux (50) du col (8), et faisant tomber l'aiguille (7) dans le corps cylindrique (1), le joint d'étanchéité (6) et la tête/support (5) ayant tous les deux au moins deux fentes hermétiques (20, 20') à travers lesquelles les petites bandes (18, 18') peuvent respectivement coulisser, de manière à empêcher une fuite de liquide quand le poussoir (2) avance ou recule dans le corps cylindrique (1).

2. Seringue jetable avec aiguille rétractable selon la revendication précédente, **caractérisée en ce que** le boîtier (10) est d'une seule pièce et est introduit au centre du joint d'étanchéité (6), et présente la forme d'un cylindre creux sur la surface intérieure duquel est prévu le filetage (30), apte à venir en prise avec le profilé circulaire (14), et **en ce que** le boîtier (10) comporte un second petit cylindre (13) en son centre.

3. Seringue jetable avec aiguille rétractable selon les revendications précédentes, **caractérisée en ce que** le support (9) de l'aiguille (7) est creux à l'intérieur, de manière à recevoir, à l'intérieur, le petit cylindre (13) dans l'extrémité ouverte (15) quand le profilé circulaire (14) est vissé sur le filetage (30), et le support (9) présentant plusieurs ailettes (12) aptes à être calées sur la face intérieure du petit cylindre creux (50) du col (8) quand le poussoir (2) est complètement poussé dans le corps cylindrique (1).

4. Seringue jetable avec aiguille rétractable selon les revendications précédentes, dans lequel l'élément de séparation (11) est disposé dans le corps cylindrique (1) de manière à être traversé, grâce au perçage (17), par les ailettes (12) et par l'aiguille (7) du support (9), et l'élément de séparation (11) étant constitué par une base discoïde (16) pourvue du perçage (17), et par au moins deux petites bandes (18, 18') qui se terminent par deux petits cylindres respectifs (19, 19') de telle sorte que lesdites petites bandes (18, 18') s'étendent le long des rainures (3) du poussoir (2).

5. Seringue jetable avec aiguille rétractable selon les revendications précédentes, **caractérisée en ce que** le col (8) du corps cylindrique (1) présente sur sa surface intérieure un filetage (40) apte à permettre le vissage d'un tube à essai stérile (100) équipé d'un filetage correspondant, de telle sorte que le petit cylindre creux (50) relié sur sa partie inférieure au col (8) et placé au centre par rapport à celui-ci, soit introduit dans le tube à essai (100) après avoir perforé la membrane qui scelle l'extrémité ouverte dudit tube.

6. Seringue jetable avec aiguille rétractable selon les revendications précédentes, **caractérisée en ce que** l'élément de séparation (11) présente un perçage (17) avec un diamètre apte à recevoir et à permettre le passage des ailettes (2) du support (9) de l'aiguille (7), tandis que le profilé circulaire (14) du support (9), qui présente un plus grand diamètre par rapport au perçage (17), restera placé au-dessous dudit perçage (17).

7. Seringue jetable avec aiguille rétractable selon les revendications précédentes, **caractérisée en ce que** les petits cylindres (19, 19') ont une taille beaucoup plus grande que les fentes (20, 20'), de sorte qu'ils ne peuvent pas passer dans lesdites fentes (20, 20').

8. Seringue jetable avec aiguille rétractable selon les revendications précédentes, comprenant un ressort (21) disposé dans le corps cylindrique (1) et au-dessus du joint d'étanchéité (6) et apte à faciliter la rétraction du poussoir (2) qui provoque le recul consécutif du joint d'étanchéité (6), qui, en entraînant les petits cylindres (19), 19'), empêchés de par leur taille de passer dans les fentes (20, 20'), provoque la libération des ailettes (12) du support (9) par rapport à l'intérieur du petit cylindre (50), faisant tomber l'aiguille (7) dans le corps cylindrique (1).

9. Seringue jetable avec aiguille rétractable selon les revendications précédentes, **caractérisée en ce que** les petites bandes (18, 18') de l'élément de séparation (11) ont une longueur plus petite que celle du corps cylindrique (1), similaire à la largeur de l'échelle graduée disposée sur le corps cylindrique (1), de telle sorte qu'en ramenant la tête/support (5) au-delà de l'extrémité de l'échelle graduée, on obtienne la séparation des ailettes (12) du support (9) par rapport à l'intérieur du petit cylindre creux (50) avant que la tête/support (5) ne sorte par l'extrémité ouverte du corps cylindrique (1).

10. Seringue jetable avec aiguille rétractable selon les revendications précédentes, **caractérisée en ce que** son petit cylindre (13) est pourvu en son centre du perçage (60), et ledit perçage (60) étant en contact, à l'aide de plusieurs perçages (70) et de conduits (71), avec le corps cylindrique (1).
